Europäisches Patentamt

European Patent Office

Office européen des brevets

(19)

(11) Publication number: **0 069 715**

**B1**

(12) **EUROPEAN PATENT SPECIFICATION**

(45) Date of publication of patent specification: **05.11.86**

(51) Int. Cl.⁴: $A\ 61\ M\ 15/00$

(21) Application number: **82850129.6**

(22) Date of filing: **10.06.82**

(54) **Powder inhalator.**

(30) Priority: **08.07.81 SE 8104239**

(43) Date of publication of application:
**12.01.83 Bulletin 83/02**

(45) Publication of the grant of the patent:
**05.11.86 Bulletin 86/45**

(84) Designated Contracting States:
**AT BE CH DE FR GB IT LI LU NL SE**

(56) References cited:
**GB-A-2 041 763**
**US-A-2 587 215**
**US-A-4 047 525**

(73) Proprietor: **Aktiebolaget Draco**
**Box 1707 Tunavägen 43**
**S-221 01 Lund (SE)**

(72) Inventor: **Wetterlin, Kjell Ingvar Leopold**
**Västerväng 19**
**S-240 17 S Sandby (SE)**

(74) Representative: **Wurm, Bengt Runio et al**
**Patent and Trade Mark Department AB ASTRA**
**S-151 85 Södertälje (SE)**

Courier Press, Leamington Spa, England.

## Description

### Field of the Invention

The present invention relates to a new dosage inhalator, intended to be activated using the air flow generated at inhalation and intended to be used for inhalation of pharmacologically active compound in solid, micronized form.

### Background of the Invention

Special requirements are made with regard to dosage inhalators intended for local administration of drugs to the respiratory tract and to the lungs. Since mostly very potent drugs are to be administered, the dose accuracy must be great. The dosage of active compound that is to be administered may be as small as 0.1 mg. It is also necessary that the particles that leave the dosage inhalator have a suitable size distribution, since too big particles tend to be deposited in the mouth.

Several systems are available for local administration of drugs to the respiratory tract and to the lungs. Among these systems may be mentioned nebulizing devices, pressurized aerosols, pump inhalators, and inhalators which are activated by the air flow generated at inhalation, herebelow denoted "powder inhalators".

Several types of powder inhalators are available on the market. They represent a complement to pressurized aerosols, where the drug is dissolved or suspended in a liquid propellant mixture. The powder inhalators have the advantage that they always deliver the active compound when the patient inhales, as the particle cloud is generated by the air flow obtained at inhalation. Thereby the problem of coordinating the activation of a dosage with the inhalation in order to bring the active compound to the respiratory tract and to the lungs is solved. An example of powder inhalators which are available on the market is Spinhaler®. In Spinhaler® the active compound is used in micronized form, contained in a hard gelatine capsule which is perforated before use. The hard gelatine capsule is placed in a tube in a device which is brought to rotation by the air flow generated at inhalation, whereby the micronized compound is moved into the air stream and is brought via the air flow to the respiratory tract and lungs of the patient.

The British patent publication GB—A—2 041 763 discloses an inhalator, which is activated by the air flow generated at inhalation and intended for free flowing pulverulent medicinal substances, wherein the active substance is measured in dosing holes and then poured onto a dispensing disc wherefrom the active substance is inhaled by the patient.

The US patent specification US—A—2 587 215 discloses an inhalator utilizing cavities in a disc as means for transporting a measured amount of free flowing powder to be inhaled from a storage space to a position from where it is inhaled by the air flow generated at inhalation.

However, the previously known powder inhalators have disadvantages:

1) they cannot be used for accurate and reproducible dispensing of micronized active compound in amounts below 15—20 mg, and they are therefor useful only for less potent active compounds or for highly potent active compounds in combination with a diluting agent, usually lactose;

2) they are cumbersome to load and to make clean;

3) usually several inhalations are required to empty a capsule containing a unit dose;

4) they are difficult to handle for patients with reduced breathing capacity or with reduced capability in the use of their hands;

5) lactose, the diluting agent, is very disturbing at inhalation and may cause increased frequence of caries.

There is a need for an effective powder inhalator which is activated by the air flow generated at inhalation, which is easy to handle for the patient, which allows dispensation of active compound in an amount down to 0.1 mg without need to include any diluting agent, and which gives a suitable size distribution for the particles which are administered.

### The Invention

The present invention relates to a dosage inhalator, a so called "powder inhalator", which is activated by the air flow generated at inhalation, and which makes it possible to dispense solid active compound in micronized form, in a suitable size distribution, in an amount from 0.1 mg without need for any diluting agent. The dosage inhalator can be constructed for administering active compound in an amount up to 5 mg. It can also, with suitable construction of the dosing unit be used for administering active compound in an amount of 5—50 mg.

The present invention relates to a powder inhalator which is activated by the air flow generated at inhalation and which is intended for inhalation of a pre-determined amount of solid pharmacologically active compound in micronized form, said inhalator comprising a nozzle, an air conduit connected to said nozzle for passage of the air to be inhaled, a dosing unit comprising a storage chamber for the active compound and a displaceable element for dispensing said compound from the storage chamber into the air conduit and a manoeuvring unit for displacing said element in relation to the storage chamber said powder inhalator being characterized in that the dispensing element is a perforated membrane, that the dosing unit comprises also a holder for the said perforated membrane, means for introducing and metering active compound into the perforations in the perforated membrane, and a plate arranged adjacent the membrane to prevent active compound from passing through the perforations in the membrane when active compound is introduced into the perforations, the said membrane being displaceably arranged in relation to said means for introducing the compound so that in a first position solid active compound in micronized form is introduced into the perforations in part of the area of the membrane and in a second position the said part of the area of the membrane including perforations holding the predetermined amount of active compound is inserted across the air conduit for the air to be inhaled.

The dosing means in combination with the storage chamber for the active compound represent the essential new elements in the powder inhalator according to the invention.

One embodiment of the dosage inhalator according to the invention is now described more in detail with reference to Figures 1, 2, and 3.

*Figure 1* is a sectional view through the dosage inhalator according to the invention.

*Figure 2* shows scrapers in the storage chamber, which scrapers are used to introduce active compound into the perforations in the perforated membrane.

*Figure 3* shows how the active compound is fed from the storage unit into the perforations in the perforated membrane using the said scrapers.

The dosage inhalator comprises a maneuvering unit 1 which is used for feeding dosages of the active compound, a nozzle 2 which may be provided with rotating means 3 intended for disrupting such aggregate particles of the active compound which might have been formed, a dosing unit 10 for measuring the intended dosage of the active compound and a storage chamber 5 for solid micronized active compound.

The dosage inhalator also comprises an air conduit 6 intended for passage of the air to be inhaled. The nozzle 2 can be provided with rotating means 3 intended for disrupting such aggregate particles of the active compound which might have been formed. The disintegration of possible particle aggregates is facilitated by air inlets 7 arranged at the side of the nozzle 2.

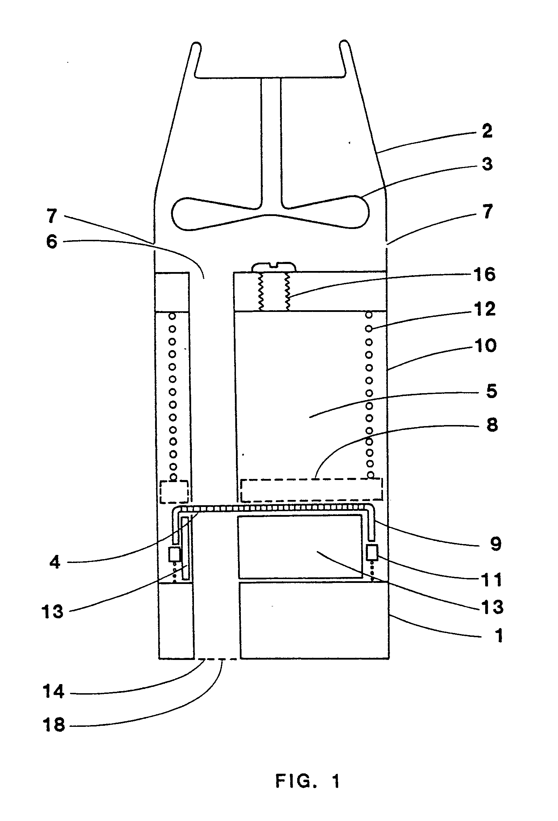

The dosing unit 10 comprises a storage chamber 5 for the active compound a perforated membrane 4, a holder 9 for the perforated membrane, and dosing means 8, schematically shown in Figure 1, for introducing the active compound into the perforations in the perforated membrane 4. The dosing unit 10 and the perforated membrane 4 are displaceably arranged in relation to one another between a first position where active compound by the dosing means 8 is introduced into the perforations in part of the area of the perforated membrane 4, and a second position where the said part area of the loaded membrane 4 has been inserted across the air conduit 6 in the dosage inhalator. In said first position of the membrane 4, the active compound is brought from the storage chamber 5 into the perforations in the membrane 4. When a part area of the membrane 4 containing such perforations filled with active compound thereafter is inserted across the air conduit 6, the active compound contained in the perforations will be entrained at inhalation and be brought through the nozzle 2 to the respiratory tract and the lungs of the patient.

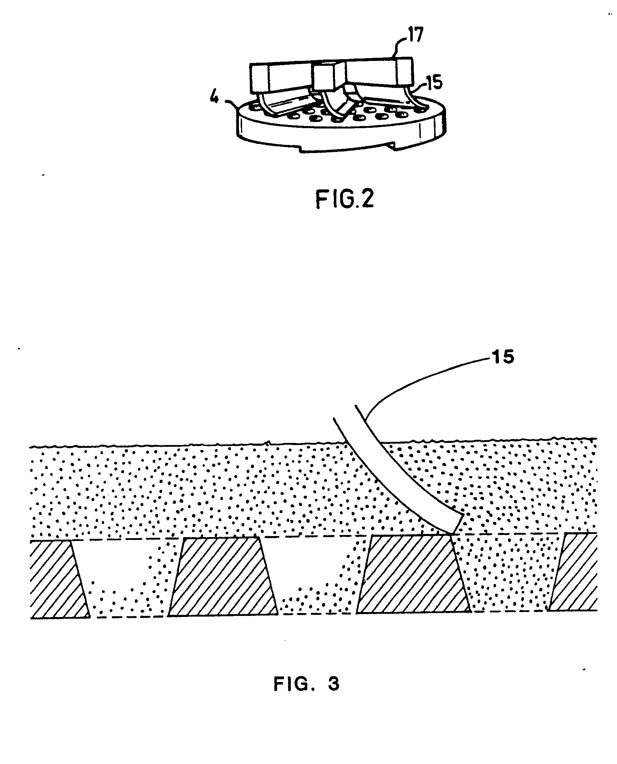

The introduction of active compound into the perforations in the perforated membrane 4 is in the described embodiment made with mechanical means 8 consisting of elastic, spring-loaded scrapers 15, mounted in a holder 17 in the storage chamber 5. See Figures 2 and 3.

In a preferred embodiment, the perforated membrane 4 is displaceably arranged in relation to the storage chamber 5.

In another preferred embodiment, the dosing unit 10 comprises a perforated membrane 4 which can be rotated and which is intended to be loaded with solid active compound in micronized form.

In a further preferred embodiment, elastic spring-loaded scrapers 15 are arranged in the storage chamber 5 to introduce active compound into the perforations in the perforated membrane 4.

In another preferred embodiment, the perforations in the perforated membrane 4 are in the form of truncated cones with their large opening directed to the nozzle.

The scrapers 15 are suitably manufactured in an elastic material, for example rubber or plastic. The scrapers are suitably arranged so that they, when the active compound is introduced into the perforations in the membrane 4, touch the surface of the membrane at an angle which is less than 90°. When the means for introducing active compound into the perforations in the membrane are arranged in this manner, the additional advantage is obtained that possible aggregates of active compound are disintegrated before administration.

The scrapers 15 are suitably arranged so that they press against the surface of the membrane 4. That is achieved as shown in Figure 1 by having the scrapers 15 loaded by a spring 12. The spring 12 is arranged in the storage chamber 5 as is shown in Figure 1.

Alternatively, a spring can be arranged so that the membrane 4 is pressed against the scrapers 15. The spring may in such case be arranged outside the storage chamber 5, suitably mounted in the maneuvering unit 1. Further variations are possible. The essential element is that the scrapers 15 press against the membrane 4 in such a way that active compound is introduced into the perforations in the membrane 4 when the maneuvering unit is operated. See Figure 2 and Figure 3.

In the maneuvering unit 1, immediately adjacent to the membrane 4, a plate 13 is arranged which prevents active compound from passing through the perforations in the membrane 4. See Figure 1. Thus, the plate 13 will comprise the bottom of the perforations.

The air conduit 6 passes through the maneuvering unit 1. At inhalation through the nozzle 2 the air flow will pass partly through the air inlets 7 arranged at the side of the nozzle 2, partly through the air conduit 6. The air in the air conduit 6 passes through its opening 14 in the maneuvering unit 1. In the embodiment shown in Figure 1, a perforated plate 18 is arranged as a filter in the opening 14 in order to prevent undesired particles of larger size from entering the air conduit 6.

The perforated membrane 4 in the dosing unit 10 can be made, as is illustrated in Figure 1 and in Figure 3, as a horizontal membrane. But also other embodiments are possible, for example a membrane in the form of a drum where the active compound is filled into the perforations from a storage chamber which can be arranged outside the drum or inside the drum.

When a membrane in the form of a horizontal plane is used, as illustrated in Figure 1, the membrane can be mounted so that it can be moved by rotating it, whereby the other parts of the dosage inhalator are fixed relative to one another.

In order to make sure that the amount of active compound that has been filled into the perforations which are inserted in the air conduit 6 is released from the perforations and entrained at inhalation, the said perforations in the membrane are suitably formed as truncated cones, see Figure 3, with the wider opening directed towards the nozzle. Perforations in the form of truncated cones facilitate the emptying of the perforations in that the active compound is released more easily. Moreover, when the storage chamber 5 is arranged between the membrane and the nozzle, also the filling of the perforations is facilitated if the perforations are designed as is shown in Figure 3.

The perforations in the perforated membranes can be of arbitrary design. They can be circular, square, elliptic, rectangular or have other geometrical form. The area of the perforations in the membrane can be a large or a small part of the membrane area, for example from 1 to 95%, whereby the term "membrane area" refers to that part of the area of the membrane which is inserted across the air conduit. The number of perforations in the membrane area can vary depending on factors such as the amount of active substance that is to be administered per dosage, the physical properties of the active substance, etc. In a preferred embodiment the perforations have conical shape.

The perforated membrane can be manufactured in any suitable material, for example metal or plastic. The size of the dosage of active compound which is to be administered is determined by the size of the perforations in the membrane, the thickness of the membrane, the number of perforations in the membrane and by the size of the air conduit. A single perforation may be sufficient for dispensing a given dose of the active compound. The accuracy of the dosage will mainly depend on the accuracy in the manufacturing of the membrane. Examples of perforated membranes that can be used are the metal nets which are manufactured by Veco Beheer B.V., Eerbeek, The Netherlands. These nets can be obtained with various sizes of the perforations. They can be formed in desired manner, for example in drum form or they can be used in the form of horizontal, plane membranes. Also woven nets of metal, fiber or of other materials can be used. The important factor is the dosage accuracy that can be obtained.

The maneuvering unit 1 is in the embodiment shown in Figure 1 arranged adjacent to the dosing unit 10. The maneuvering unit can be made in the form of a dented ring. Spring-loaded pins 11, see Figure 1, are used to provide distinct positions for the perforated membrane when it is advanced by operating the maneuvering unit 1. The maneuvering unit can be arranged also otherwise, for example by arranging it to operate directly on the perforated membrane.

The storage chamber is in a preferred embodiment arranged between the perforated membrane and the nozzle. See Figure 1.

The storage chamber can be arranged to accommodate a varying amount of the active compound. In a dosage inhalator where the storage chamber is not made for refilling of active compound it can contain an amount of active compound corresponding for example up to about 200 dosage units. In a dosage inhalator where the storage chamber is intended for refilling a device is required which can be arranged on the top or on the sides of the storage chamber. For example, a screw or plug 16 can be arranged in the storage chamber 5 as is shown in Figure 1.

The air conduit 6 can have an area of 25—350 $mm^2$. The air conduit can be circular or have other geometrical form. If it is circular, the diameter may be from 3 to 10 mm.

Among compound groups and specific compounds which are suitable for administering with a powder inhalator according to the present invention can be mentioned

— betareceptorstimulating agents such as adrenaline, isoprenaline, orciprenaline, salbutamol and terbutaline,

— steroids for inhalation such as budesonide,

— substances intended for nasal administration.

Especially useful are terbutaline and budesonids.

The active compound can be administered in micronized form without additional ingredients or in pharmaceutically modified micronized form in order to obtain improved flow properties. The micronized particles may be covered with a film functioning for example by masking bitter taste of the active compound, or by providing slow release of the active compound in the respiratory tract.

In an additional aspect, the present invention relates to the use in powder inhalator of a perforated membrane as metering and holding means for solid micronized active compound.

**Claims**

1. A powder inhalator which is activated by the air flow generated at inhalation and which is intended for inhalation of a pre-determined amount of solid pharmacologically active compound in micronized form, said inhalator comprising a nozzle (2), an air conduit (6) connected to said nozzle for passage of the air to be inhaled, a dosing unit (10) comprising a storage chamber (5)

for the active compound and a displaceable element (4) for dispensing said compound from the storage chamber into the air conduit (6) and a manoeuvring unit (1) for displacing said element (4) in relation to the storage chamber said inhalator being characterised in that the dispensing element (4) is a perforated membrane, and in that the dosing unit (10) comprises also a holder (9) for the said perforated membrane, means (8) for introducing and metering active compound into the perforations in the perforated membrane (4), and a plate (13) arranged adjacent the membrane (4) to prevent active compound from passing through the perforations in the membrane (4) when active compound is introduced into the perforations, the said membrane (4) being displaceably arranged in relation to said means (8) for introducing the compound so that in a first position solid active compound in micronized form is introduced into the perforations in part of the area of the membrane (4) and in a second position the said part of the area of the membrane (4) including perforations holding the pre-determined amount of active compound is inserted across the air conduit (6) for the air to be inhaled.

2. A dosage inhalator according to claim 1, characterized in that the perforated membrane (4) can be rotated.

3. A dosage inhalator according to claim 2, characterized in that elastic spring-loaded scrapers (15) are arranged in the storage chamber (5).

4. A dosage inhalator according to claim 2, characterized in that the perforated membrane (4) is pressed against scrapers (15) with a spring mounted in the manoeuvring unit (1).

5. A dosage inhalator according to claims 2, 3, or 4, characterized in that the perforations in the perforated membrane (4) are in the form of truncated cones with their large opening directed to the nozzle (2).

6. A dosage inhalator according to any of claims 1—5, adapted for administration of a dosage of 0.1—5 mg of the active compound.

7. A dosage inhalator according to any of claims 1—5, adapted for administration of a dosage of 5—50 mg of the active compound.

8. The use in powder inhalators of a displaceable perforated membrane as metering and transporting means for solid micronized active compound in a pre-determined amount of 0.1—5 mg.

9. The use in powder inhalators of a displaceable perforated membrane as metering and transporting means for solid micronized active compound in a pre-determined amount of 5—50 mg.

**Patentansprüche**

1. Pulverinhalator, der durch den bei der Inhalation erzeugten Luftstrom aktiviert wird und zur Inhalation einer vorbestimmten Menge einer festen pharmakologisch aktiven Verbindung in pulverisierter Form bestimmt ist, welcher Inhalator eine Düse (2), eine mit der Düse zwecks Durchtritts der zu inhalierenden Luft verbundene Luftleitung (6), eine eine Speicherkammer (5) für die aktive Verbindung und ein verlagerbares Element (4) zur Abgabe der Verbindung von der Speicherkammer in die Luftleitung (6) und eine Manövriereinheit (1) zum Verlagern des Elements (4) in bezug auf die Speicherkammer umfassende Dosierungseinheit (10) umfaßt, welcher Inhalator dadurch gekennzeichnet, ist, daß das Abgabeelement (4) eine perforierte Membran ist und daß die Dosierungseinheit auch einen Halter (9) für die perforierte Membran, Mittel (8) zur Einführung und Dosierung der aktiven Verbindung in die Perforationen in der perforierten Membran (4) und eine angrenzend an die Membran (4) angeordnete Platte (13) zur Verhinderung des Durchtritts von aktiver Verbindung durch die Perforationen in der Membran (4), wenn aktive Verbindung in die Perforationen eingebracht ist, umfaßt, wobei die Membran (4) in bezug auf diese Mittel (8) zur Einführung der Verbindung verlagerbar ist, sodaß in einer ersten Position feste aktive Verbindung in pulverisierter Form in die Perforationen in einem Teil der Fläche der Membran (4) eingeführt und in einer zweiten Position dieser Teil der Fläche der Membran (4) mit den Perforationen, die die vorbestimmte Menge der aktiven Verbindung halten, quer über die Luftleitung (6) für die zu inhalierende Luft eingesetzt wird.

2. Dosierungsinhalator nach Anspruch 1, dadurch gekennzeichnet, daß die perforierte Membran (4) drehbar ist.

3. Dosierungsinhalator nach Anspruch 2, dadurch gekennzeichnet, daß elastische, federbeaufschlagte Schaber (15) in der Speicherkammer (5) angeordnet sind.

4. Dosierungsinhalator nach Anspruch 2, dadurch gekennzeichnet, daß die perforierte Membran (4) mit einer in der Manövriereinheit (1) montierten Feder gegen Schaber (15) gepreßt wird.

5. Dosierungsinhalator nach den Ansprüchen 2, 3 oder 4, dadurch gekennzeichnet, daß die Perforationen in der perforierten Membran (4) die Form von Kegelstümpfen haben, wobei ihre große Öffnung zur Düse (2) gerichtet ist.

6. Dosierungsinhalator nach einem der Ansprüche 1—5, ausgelegt zur Verabreichung einer Dosis von 0,1—5 mg der aktiven Verbindung.

7. Dosierungsinhalator nach einem der Ansprüche 1—5, ausgelegt zur Verabreichung einer Dosis von 5—50 mg der aktiven Verbindung.

8. Verwendung einer verlagerbaren perforierten Membran als Dosier- und Transportmittel für eine feste, pulverisierte aktive Verbindung in einer vorbestimmten Menge von 0,1—5 mg in Pulverinhalatoren.

9. Verwendung einer verlagerbaren perforierten Membran als Dosier- und Transportmittel für eine feste, pulverisierte aktive Verbindung in einer vorbestimmten Menge von 5—50 mg in Pulverinhalatoren.

**Revendications**

1. Un inhalateur de poudre qui est actionné par le courant d'air engendré au cours de l'inhalation et qui est destiné pour l'inhalation d'une quantité prédéterminée d'un composé actif, solide, pharmacologiquement sous forme micronisée, ledit inhalateur comprenant une buse (2), un conduit d'air (6) relié à ladite buse pour le passage de l'air à inhaler, une unité de dosage (10) comprenant une chambre d'emmagasinage (5) pour le composé actif et un élément pouvant être déplacé (4) pour distribuer ledit composé à partir de la chambre d'emmagasinage à l'intérieur d'un conduit d'air (6) et une unité de manoeuvre (1) pour déplacer ledit élément (4) par rapport à la chambre d'emmagasinage, ledit inhalateur étant caractérisé en ce que l'élément de distribution (4) est une membrane perforée, et en ce que l'unité de dosage (10) comprend aussi un support (9) pour ladite membrane perforée, des moyens (8) pour introduire et doser le composé actif à l'intérieur des perforations ménagées dans la membrane perforée (4), et une plaque (13) disposée en position adjacente à la membrane (4) pour empêcher le composé actif de traverser les perforations ménagées dans la membrane (4) lorsque le composé actif est introduit dans les perforations, ladite membrane (4) pouvant être déplacée par rapport auxdits moyens (8) pour introduire le composé, de telle sorte que dans une première position le composé actif solide sous forme micronisée est introduit dans les perforations sur une partie de la surface de la membrane (4) et dans une seconde position ladite partie de la membrane (4) comportant les perforations contenant la quantité prédéterminée de composé actif est insérée dans le conduit d'air (6) par l'air à inhaler.

2. Un inhalateur de dosage selon la revendication 1, caractérisé en ce que la membrane perforée (4) peut être entraînée en rotation.

3. Un inhalateur de dosage selon la revendication 2, caractérisé en ce que les raclettes (15) élastiques chargées par ressort sont disposées dans la chambre d'emmagasinage (5).

4. Un inhalateur de dosage, selon la revendication 2, caractérisé en ce que la membrane perforée (4) est poussée contre les raclettes (15) par un ressort monté dans l'unité de manoeuvre (1).

5. Un inhalateur de dosage selon les revendications 2, 3 ou 4, caractérisé en ce que les perforations ménagées dans la membrane perforée (4) sont sous forme de tronc de cône, avec leur ouverture large dirigée vers la buse (2).

6. Un inhalateur de dosage selon l'une quelconque de revendications 1—5, destiné à l'administration d'une dose de 0,1—5 mg de composé actif.

7. Un inhalateur de dosage selon l'une quelconque des revendications 1—5, destiné à l'administration d'une dose de 5—50 mg de composé actif.

8. L'utilisation dans les inhalateurs de poudre d'une membrane perforée pouvant être déplacée comme moyen de dosage et de transport pour un composé actif micronisé en quantité prédéterminée, de 0,1—5 mg.

9. L'utilisation dans les inhalateurs de poudre d'une membrane perforée pouvant être déplacée comme moyen de dosage et de transport pour un composé actif solide micronisé, dans une quantité prédéterminée de 5—50 mg.

FIG. 1

FIG.2

FIG. 3